# EUROPÄISCHE PATENTSCHRIFT

(11) **EP 1 491 186 B1**
(45) Veröffentlichungstag und Bekanntmachung des Hinweises auf die Patenterteilung: **11.08.2010**
(21) Anmeldenummer: 04015087.2
(22) Anmeldetag: 28.06.2004
(51) Int. Cl.: A61K 8/02, A61K 8/06, A61K 8/37, A61Q 19/00, A61Q 19/10

(54) **Inhomogen aufgebauter Reinigungsartikel**
Cleaning article of heterogeneous constitution
Article de toilette de constitution hétérogène

(30) Priorität: 26.06.2003 DE 10329210
(43) Veröffentlichungstag der Anmeldung: 29.12.2004
(73) Patentinhaber: Beiersdorf AG, 20253 Hamburg (DE)
(72) Erfinder: Kröpke, Rainer, 22869 Schenefeld (DE); von der Fecht, Stephanie, 22869 Schenefeld (DE); Hahn, Ingo, 25421 Pinneberg (DE)

(56) Entgegenhaltungen:
- WO-A-02/36339
- WO-A-94/02674
- US-A- 4 242 405
- N. OLTARZHEVSKAYA ET AL.: "Textile materials for local treatment of gun wounds" TEXTILNAYA KHIMIYA, März 2003 (2003-03), Seiten 53-58, XP008048102 RU
- DATABASE CA CHEMICAL ABSTRACTS SERVICE, COLUMBUS, OHIO, US; XP002330959 gefunden im STN Database accession no. 54: 113 442 & J. KIGER: "surgical pads from hydrophilic viscose" ANNALES PHARMACEUTIQUES FRANÇAISES, Bd. 18, 1960, Seiten 192-205, FR
- A. WATZL ET AL.: "spunlaced and airlaid nonwovens for medical textiles" COLOURAGE ( TAI GOLDEN JUBILEE CONFERENCE 14-16 DEC.2002), 2002, Seiten 55-62, XP008048040
- C. SUN ET AL.: "development of innovative cotton-surfaced nonwoven laminates" JOURNAL OF INDUSTRIAL TEXTILES, Bd. 31, Nr. 3, 2002, Seiten 179-188, XP008048041

## Beschreibung

Die vorliegende Erfindung betrifft einen kosmetischen und/oder dermatologischen Reinigungsartikel.

Die Produktion von kosmetischen Reinigungsmitteln zeigt seit Jahren eine steigende Tendenz. Dies ist vor allem auf das zunehmende Gesundheitsbewußtsein und Hygienebedürfnis der Verbraucher zurückzuführen.

Reinigung des menschlichen Körpers bedeutet das Entfernen von (Umwelt-) Schmutz und bewirkt damit eine Erhöhung des psychischen und physischen Wohlbefindens. Die Reinigung der Oberfläche von Haut und Haaren ist ein sehr komplexer, von vielen Parametern abhängiger Vorgang. Zum einen sollen von außen kommende Substanzen wie beispielsweise Kohlenwasserstoffe oder anorganische Pigmente aus unterschiedlichsten Umfeldern sowie Rückstände von Kosmetika oder auch unerwünschte Mikroorganismen möglichst vollständig entfernt werden. Zum anderen sind körpereigene Ausscheidungen wie Schweiß, Sebum, Haut- und Haarschuppen ohne tiefgreifende Eingriffe in das physiologische Gleichgewicht der Haut abzuwaschen.

Eine besondere Produktform für Reinigungszubereitungen stellen feste Reinigungssubstrate bzw. -textilien dar, insbesondere Tücher. Diese können bereits vom Hersteller mit der Reinigungszubereitung getränkt sein (eine Kombination, für die im Rahmen der vorliegenden Erfindung auch der Begriff "Reinigungsartikel" verwendet wird) und h aben d adurch d en V orteil, d ass in ihnen d ie Z ubereitung b ereits in d er r ichtigen Dosierung vorgegeben ist. Außerdem vermeiden sie den Nachteil von in Flaschen aufbewahrten Zubereitungen, deren Verpackung zerbrechen und deren Inhalt "auslaufen" kann. Zu den weiteren Vorteilen von Reinigungssubstraten/Textilien zählen auch die Umstände, dass sie sich bequem in abgezählter Menge mit auf Reisen nehmen lassen und für ihre Anwendung in der Regel kein Wasser mehr erforderlich ist.

Reinigungssubstrate/Tücher werden a us T extilien hergestellt. Textilien können g ewebt, gestrickt oder gewirkt sein oder als Verbundstoff (engl. nonwoven textile) vorliegen. Meist werden (aus Kostengründen) Verbundstoffe verwendet. Bei Verbundstoffen erfolgt die Gewebebildung nicht durch Kette und Schuss oder Maschenbildung, sondern durch Verschlingung, und/oder kohäsive und/oder adhäsive Verbindung von Textilfasern. Verbundstoffe können nach der DIN 61210 T2 in Vlies, Papier Watte und Filz unterschieden werden. Vliese sind lockere Materialien aus Spinnfasern (d.h. Faser mit begrenzter Länge) oder Filamenten (Endlosfasern), meist aus Polypropylen, Polyester oder Viskose hergestellt, deren Zusammenhalt im allgemeinen durch die den Fasern eigene Haftung gegeben ist. Hierbei können die Einzelfasern eine Vorzugsrichtung aufweisen (orientierte oder Kreuzlage-Vliese) oder ungerichtet (Wirrvliese) sein. Die Vliese können mechanisch verfestigt werden durch Vernadeln, Vermaschen oder durch Verwirbeln mittels scharfer Wasserstrahlen. Adhäsiv verfestigte Vliese entstehen durch Verkleben der Fasern mit flüssigen Bindemitteln (z.B. Acrylat-Polymere, SBR/NBR, Polyvinylester, Polyurethan-Dispersionen) oder durch Schmelzen oder Auflösen von sogenannten Bindefasern, die dem Vlies bei der Herstellung beigemischt wurden. Bei der kohäsiven Verfestigung werden die Faseroberflächen durch geeignete Chemikalien angelöst und durch Druck verbunden oder bei erhöhter Temperatur verschweißt [J. Falbe, M. Regnitz: Römpp-Chemie-Lexikon, 9. Aufl. Thieme-Verlag, Stuttgart (1992)].

Mit kosmetischen Zubereitungen imprägnierte Substrate und insbesondere Tücher können auf unterschiedlichen Wegen hergestellt werden: Im sogenannten "TauchVerfahren" wird das Tuch in einem Tauchbad eingetaucht oder durch ein Bad gezogen. Dieses Verfahren eignet sich insbesondere für Papiertücher und weniger für Vliese, da letztere zu viel Flüssigkeit (=Zubereitung) aufnehmen und sich in Umverpackung anschließend Pfützen von wieder freigesetzter Zubereitung finden.

Eine zweite Variante stellt das "Sprüh-Verfahren" dar, bei dem die Zubereitung auf das vorbeilaufende Tuch aufgesprüht wird. Diese Verfahren eignet sich für alle Textilien, doch können keine stark schäumenden Zubereitungen auf das Tuch aufgebracht werden, da die Schaumentwicklung beim Sprühverfahren zu groß wird.

Als weitere Methode kommen sogenannte Abstreifmethoden zum Einsatz. Dort laufen Vlies oder Tuchbahnen an Abstreifblechen, -balken oder -düsen vorbei, die kontinuierlich mit Imprägnierungslösung beladen werden. Unterschiedliche Imprägnierungsgrade lassen sich u. a. durch Variation des Anpressdruckes und der Tuchzuggeschwindigkeit einstellen.

Nach dem Stande der Technik hergestellte Substrate/Tücher, die mit Reinigungszubereitungen oder anderen kosmetischen Zubereitungen imprägniert sind (= Reinigungsartikel), haben jedoch eine Reihe von Nachteilen:
■ Bei sogenannten "feuchten" Tüchern kommt es, insbesondere wenn sich mehrere Tücher zusammen in einem Verpackungsbehältnis befinden, bei der Lagerung zu einer Anreicherung der kosmetischen Zubereitung am Boden der Verpackung. Die in der Verpackung oben auf liegenden Tücher sind meist relativ trocken, während sich die Tücher, welche sich am Packungsboden befinden, stark mit der Zubereitung vollgesogen haben. Dieses Phänomen tritt besonders stark bei dünnflüssigen Zubereitungen auf.
■ Herkömmliche Tücher können, aufgrund ihres geringen Quellvermögens, nur geringe Mengen an kosmetischer Zubereitung aufnehmen und speichern ohne dass es zu einem "Durchsickern" und Anreichern der Zubereitung auf dem Packungsboden kommt. Darunter leidet die (Reinigungs-) Leistung des Tuches, da weniger Zubereitung an die Haut abgegeben werden und ebenfalls weniger Verunreinigungen von der Haut durch das Tuch aufgenommen werden können.

Insbesondere offenbart die Patentanmeldung WO 94/02674 Reinigungsartikel, die Baumwollfasern enthalten können.

Es war daher die Aufgabe der vorliegenden Erfindung, die Mängel des Standes der Technik zu beseitigen und Reinigungsartikel (d.h. Kombinationen aus Reinigungssubstrat und kosmetischer Zubereitung) zu entwickeln, die eine hohe Saug- und Quellfähigkeit sowie eine hohe Speicherfähigkeit gegenüber flüssigen Zubereitungen aufweisen.

Überraschend gelöst wird die Aufgabe durch kosmetische Reinigungsartikel aus
a) einem Textil welches gebildet wird aus
   1-30 Gewichts-% Baumwollfasern,
   9-80 Gewichts-% Viskosefasern und
   19-90 Gewichts-% Polyester, jeweils bezogen auf das Gesamtgewicht des Textils, welches dadurch gekennzeichnet ist, dass der Gehalt an Baumwollfasern im Textil zur Textiloberfläche hin zunimmt und -
b) einer kosmetischen Zubereitung welche gewählt wird aus der Gruppe der Öle, Silikonöle, Emulsionen, Dispersionen, alkoholischen Lösungen und wässrigen Lösungen.

Die erfindungsgemäßen Reinigungsartikel weisen eine überraschend große Saugkraft auf. Sie halten auch im gestapelten Zustand die aufgesogene kosmetische Zubereitung, ohne dass die Zubereitung nach unten durchsickert und sich in den unteren Textilien (bzw. Textilschichten) anreichert. Dabei geben sie bei der Anwendung die in der Zubereitung enthaltenen Wirk- und Pflegestoffe überraschend leicht ab, da sich diese überraschend stark in den baumwollreichen Oberflächen des Textils anreichern. Ferner war es für den Fachmann nicht vorher zu sehen, dass sich die Textilien trotz der höheren Beladung mit kosmetischer Zubereitung trockener und weicher anfühlen als herkömmliche Textilien des Standes der Technik. Insgesamt weisen die erfindungsgemäßen Reinigungsartikel eine deutlich höhere Reinigungsleistung auf als Reinigungsartikel des Standes der Technik.

Es ist erfindungsgemäß vorteilhaft, wenn es sich bei dem erfindungsgemäßen Textil um ein Vlies und besonders bevorzugt um ein Tuch oder ein sogenanntes "Pad" handelt.

Erfindungsgemäß bevorzugt werden Textilien eingesetzt, welche aus Vlies bestehen, insbesondere aus wasserstrahlverfestigten und/oder wasserstrahlgeprägten Vlies.

Erfindungsgemäß vorteilhaft weist das erfindungsgemäße Textil an der Oberfläche einen Baumwollanteil bis 30 Gewichts-% und im Inneren einen Baumwollanteil bis 10 Gewichts-%, jeweils bezogen auf das Gesamtgewicht des Textils, auf.

Die erfindungsgemäßen Textilien können glatt oder auch oberflächenstrukturiert (beispielsweise genoppt oder gelocht) sein. Erfindungsgemäß bevorzugt sind oberflächenstrukturierte Textilien.

Derartige Textilien können Makroprägungen jeden gewünschten Musters aufweisen. Die zu treffende Auswahl richtet sich zum einen nach der aufzubringenden Tränkung und zum anderen nach dem Einsatzfeld, auf dem das spätere Textil Verwendung finden soll.

Werden geprägte Vliese verwendet, so erleichtern große Kavitäten an der Vliesoberfläche und im Vlies die Aufnahme von Schmutz und Verunreinigungen, wenn mit dem getränkten Tuch über die Haut gefahren wird. Die Reinigungswirkung kann gegenüber ungeprägten Tüchern um ein Vielfaches gesteigert werden.

Es hat sich als vorteilhaft herausgestellt für das Tuch, wenn dieses ein Gewicht von 35 bis 120 g/m², vorzugsweise von 40 bis 60 g/m², hat (gemessen bei 20 °C ± 2 °C und bei einer Feuchtigkeit der Raumluft von 65 % ± 5 % für 24 Stunden).

Die durchschnittliche Dicke des Vlieses beträgt vorzugsweise 0,4 mm bis 2 mm, insbesondere 0,6 mm bis 1,2 mm (gemessen nach der Methode ERT 30.5-99).

Als Ausgangsmaterialien für den Vliesstoff des Textils können neben den erfindungsgemäßen Faserstoffen generell alle organischen und anorganischen Faserstoffe auf natürlicher und synthetischer Basis verwendet werden. Beispielhaft seien Zellulose, Jute, Hanf, Sisal, Seide, Wolle, Polypropylen, Polyethylenterephthalat (PET), Aramid, Nylon, Polyvinylderivate, Polyurethane, Polylactid, Polyhydroxyalkanoat, Celluloseester und/oder Polyethylen sowie auch mineralische Fasern wie Glasfasern oder Kohlenstofffasern angeführt. Die vorliegende Erfindung ist aber nicht auf die genannten Materialien beschränkt, sondern es können eine Vielzahl weiterer Fasern zur Vliesbildung eingesetzt werden. Es ist insbesondere vorteilhaft im Sinne der vorliegenden Erfindung, wenn die eingesetzten Fasern nicht wasserlöslich sind.
Darüber hinaus können die Fasern auch eingefärbt sein, um die optische Attraktivität des Vlieses betonen und/oder erhöhen zu können. Die Fasern können zusätzlich UV-Stabilisatoren und/oder Konservierungsmittel enthalten.

Die zur Bildung des Tuches eingesetzten Fasern weisen vorzugsweise eine Wasseraufnahmerate von mehr als >20 mm/[10 min] (gemessen mit dem EDANA Test 10.2-96), auf.

Ferner weisen die zur Bildung des Tuches eingesetzten Fasern vorzugsweise ein Wasseraufnahmevermögen von mehr als >9 g/g (gemessen mit dem EDANA Test 10.2-96), auf.

Vorteilhafte Tücher im Sinne der vorliegenden Erfindung haben eine Reißkraft von insbesondere (gemessen nach der Methode ERT 20.2-89)

| | | [N/50mm] |
|---|---|---|
| im trockenen Zustand | Maschinenrichtung | >70, vorzugsweise >80 |
| | Querrichtung | >28, vorzugsweise >30 |
| im getränkten Zustand | Maschinenrichtung | >50, vorzugsweise >60 |
| | Querrichtung | >24, vorzugsweise >30 |

Die Dehnfähigkeit vorteilhafter Tuches beträgt vorzugsweise (gemessen nach der Methode ERT 20.2-89)

| | | |
|---|---|---|
| im trockenen Zustand | Maschinenrichtung | 45 +/- 15% |
| | Querrichtung | 110 +/- 20% |
| im getränkten Zustand | Maschinenrichtung | 45 +/- 15% |
| | Querrichtung | 90 +/- 20% |

Erfindungsgemäß vorteilhaft beträgt der Tränkungsgrad des Reinigungsartikels, d.h. das Gewichtsverhältnis von kosmetischer Zubereitung (=flüssiger Tränkung) und Textil, von 2,1 bis 4,0 , bevorzugt von 2,4 bis 3,7 und besonders bevorzugt von 2,7 bis 3,4.

Erfindungsgemäß vorteilhafte Reinigungsartikel sind dadurch gekennzeichnet, dass es sich bei der kosmetischen Zubereitung um ein Öl handelt, welches aus flüssigem Paraffin, Mineralöl, Cyclomethicon, Dimethicon, Silikongummi, Phenyltrimethylmethicon, Cariinsäure/Caprylsäure Triglycerid, C₁₂-C₁₅Alkylbenzoaten, Sheabutter, Butylenglycoldicaprat/-dicaprylat, Dicaprylylcarbonat, Octyldodecanol oder deren Mischungen gebildet wird.

Andere erfindungsgemäß vorteilhafte Reinigungsartikel sind dadurch gekennzeichnet, dass es sich bei der kosmetischen Zubereitung um eine Emulsion handelt, die Glycerylstearat und/oder Glycerylisostearat als Emulgator enthält.

Ferner handelt es sich um erfindungsgemäß vorteilhafte Reinigungsartikel, wenn diese dadurch gekennzeichnet sind, dass es sich bei der kosmetischen Zubereitung um eine Hydrodispersion handelt,

Erfindungsgemäß vorteilhaft sind Reinigungsartikel, die dadurch gekennzeichnet sind, dass es sich bei der kosmetischen Zubereitung um eine alkoholische Lösung handelt, die mindestens 5 Gewichts-%, bezogen auf das Gesamtgewicht der Zubereitung, Ethanol enthält.

Auch handelt es sich erfindungsgemäß um vorteilhafte Ausführungsformen, wenn der erfindungsgemäße R einigungsartikel d adurch gekennzeichnet i st, d ass es sich bei d er kosmetischen Zubereitung um eine wässrige Lösung handelt, die Butylenglycol oder weitere Befeuchtungsmittel enthält.

Derartige erfindungsgemäße Reinigungsartikel können darüber hinaus erfindungsgemäß vorteilhaft einen oder mehrere kosmetische oder dermatologische Wirk-, Hilfs- und Zusatzstoffe enthalten.

Eine erfindungsgemäße wässrige Phase kann neben Wasser erfindungsgemäß auch andere Inhaltsstoffe enthalten, beispielsweise Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Eine erfindungsgemäße lipophile Phase der kosmetischen und/oder dermatologischen Zubereitung kann erfindungsgemäß vorteilhaft einen oder mehrere lipophile Inhaltsstoffe enthalten, die gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder u nverzweigten Alkoholen e iner K ettenlänge von 3 b is 3 0 C -Atomen, a us d er Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner können Bestandteile einer lipophilen Phase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, z. B. O livenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der lipophilen Phase einzusetzen.

Besonders vorteilhaft sind auch Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann eine lipophile Phase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Lipidphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Bestandteile der lipophilen Phase werden ferner vorteilhaft aus der Gruppe der Phospholipide gewählt. Die Phospholipide sind Phosphorsäureester acylierter Glycerine. Von größter Bedeutung unter den Phosphatidylcholinen sind beispielsweise die Lecithine, welche sich durch die allgemeine Struktur 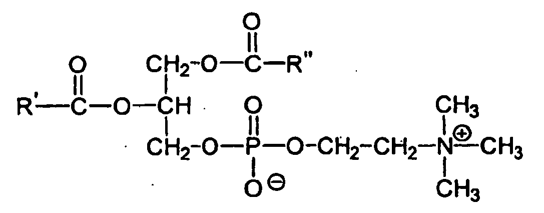 auszeichnen, wobei R' und R" typischerweise unverzweigte aliphatische Reste mit 15 oder 17 Kohlenstoffatomen und bis zu 4 cis-Doppelbindungen darstellen.

Die erfindungsgemäßen Zubereitungen können vorteilhafter Weise Verdickungsmittel enthalten. Diese Verdickungsmittel können vorteilhaft gewählt werden aus der Gruppe der Gummen, Polysaccharide, Cellulosederivate, Schichtsilikate, Polyacrylate und/oder anderen Polymeren.

Zu den Gummen zählt man Pflanzen- oder Baumsäfte, die an der Luft erhärten und Harze bilden oder Extrakte aus Wasserpflanzen. Aus dieser Gruppe können vorteilhaft im Sinne der vorliegenden Erfindung gewählt werden beispielsweise Gummi Arabicum, Johannisbrotmehl, Tragacanth, Karaya, Guar Gummi, Pektin, Gellan Gummi, Carrageen, Agar, Algine, Chondrus, Xanthan Gummi.

Weiterhin vorteilhaft ist die Verwendung von derivatisierten Gummen wie z.B. Hydroxypropyl Guar (Jaguar® HP 8).

Unter den Polysacchariden und -derivaten befinden sich z.B. Hyaluronsäure, Chitin und Chitosan, Chondroitinsulfate, Stärke und Stärkederivate.

Unter den Cellulosederivaten befinden sich z.B. Methylcellulose, Carboxymethylcellulose, Hydroxyethylcellulose, Hydroxypropylmethylcellulose.

Unter den Schichtsilikaten befinden sich natürlich vorkommende und synthetische Tonerden wie z.B. Montmorillonit, Bentonit, Hektorit, Laponit, Magnesiumaluminiumsilikate wie Veegum®. Diese können als solche oder in modifizierter Form verwendet werden wie z.B. Stearylalkonium Hektorite.

Weiterhin können vorteilhaft auch Kieselsäuregele verwendet werden.

Unter d en erfindungsgemäßen P olymeren b efinden s ich z.B. P olyacrylamide (Seppigel 305), Polyvinylalkohole, PVP, PVP / VA Copolymere, Polyglycole.
Erfindungsgemäß vorteilhafte Polyacrylate sind Polymere der Acrylsäure, insbesondere solche, die aus der Gruppe der sogenannten Carbomere oder Carbopole (Carbopol® ist eigentlich eine eingetragene Marke der B. F. Goodrich Company) gewählt werden. Polyacrylate sind Verbindungen der allgemeinen Strukturformel 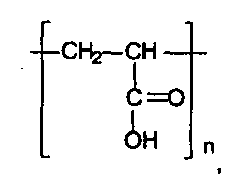 deren Molgewicht zwischen ca. 400 000 und mehr als 4 000 000 betragen kann. In die Gruppe der Polyacrylate gehören ferner Acrylat-Alkylacrylat-Copolymere, beispielsweise solche, die sich durch die folgende Struktur auszeichnen:

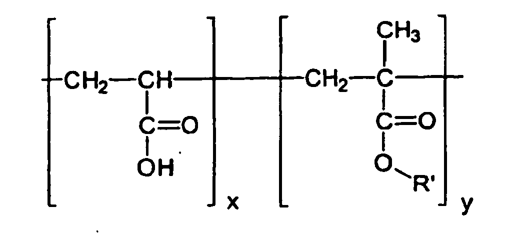

Darin stellen R' einen langkettigen Alkylrest und x und y Zahlen dar, welche den jeweiligen stöchiometrischen Anteil der jeweiligen Comonomere symbolisieren. Auch diese Polyacrylate sind vorteilhaft im Sinne der vorliegenden Erfindung.

Vorteilhafte Carbopole sind beispielsweise die Typen 907, 910, 934, 940, 941, 951, 954, 980, 981, 1342, 1382, 2984 und 5984 oder auch die Typen ETD (Easy-to-disperse) 2001, 2020, 2050, wobei diese Verbindungen einzeln oder in beliebigen Kombinationen untereinander vorliegen können.

Besonders bevorzugt sind Carbopol 981, 1382 und ETD 2020 (sowohl einzeln als auch in Kombination).

Ferner vorteilhaft im Sinne der vorliegenden Erfindung sind die den Acrylat-Alkylacrylat-Copolymeren vergleichbaren Copolymere aus C₁₀₋₃₀-Alkylacrylaten und einem oder mehreren Monomeren der Acrylsäure, der Methacrylsäure oder deren Ester. Die INCl-Bezeichnung für solche Verbindungen ist "Acrylates/C 10-30 Alkyl Acrylate Crosspolymer". Insbesondere vorteilhaft sind die unter den Handelsbezeichnungen Pemulen TR1, Pemulen TR2, Carbopol 1382 und Carbopol ETD 2020 bei der B. F. Goodrich Company erhältlichen.

Die Zusammensetzungen enthalten gemäß der Erfindung außer den vorgenannten Substanzen gegebenenfalls die in der Kosmetik üblichen Zusatzstoffe, beispielsweise P arfüm, Farbstoffe, antimikrobielle Stoffe, rückfettende Agentien, Komplexierungs- und Sequestrierungsagentien, Perlglanzagentien, Pflanzenextrakte, Vitamine, Wirkstoffe, Konservierungsmittel, Bakterizide, Pigmente, die eine färbende Wirkung haben, weichmachende, anfeuchtende und/oder feuchthaltende Substanzen, oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

Ein zusätzlicher Gehalt an Antioxidantien ist im allgemeinen bevorzugt. Erfindungsgemäß können als günstige A ntioxidantien alle f ür kosmetische u nd/oder d ermatologische A n-wendungen geeigneten oder gebräuchlichen Antioxidantien verwendet werden.

Vorteilhaft ist es auch, die Wirkstoffe aus der Gruppe der rückfettenden Substanzen zu wählen, beispielsweise Purcellinöl, Eucerit® und Neocerit® .

Die erfindungsgemäßen Emulsionen können erfindungsgemäß vorteilhaft geringe Mengen an für Lebensmittel zugelassenen Konservierungsstoffen enthalten. In der Lebensmitteltechnologie zugelassene Konservierungsmittel, welche auch vorteilhaft im Sinne der vorliegenden Erfindung eingesetzt werden können, sind mit ihrer E-Nummer nachfolgend aufgeführt.

| | | | |
|---|---|---|---|
| E 200 | Sorbinsäure | E 227 | Calciumhydrogensulfit |
| E 201 | Natriumsorbat | E 228 | Kaliumhydrogensulfit) |
| E 202 | Kaliumsorbat | E 230 | Biphenyl (Diphenyl) |
| E 203 | Calciumsorbat | E 231 | Orthophenylphenol |
| E 210 | Benzoesäure | E 232 | Natriumorthophenylphenolat |
| E 211 | Natriumbenzoat | E 233 | Thiabendazol |
| E 212 | Kaliumbenzoat | E 235 | Natamycin |
| E 213 | Calciumbenzoat | E 236 | Ameisensäure |
| E 214 | p-Hydroxybenzoesäureethylester | E 237 | Natriumformiat |
| E 215 | p-Hydroxybenzoesäureethylester-Na-Salz | E 238 | Calciumformiat |
| E 216 | p-Hydroxybenzoesäure-n-propylester | E 239 | Hexamethylentetramin |
| E 217 | p-Hydroxybenzoesäure-n-propylester-Na-Salz | E 249 | Kaliumnitrit |
| E 218 | p-Hydroxybenzoesäuremethylester | E 250 | Natriumnitrit |
| E 219 | p-Hydroxybenzoesäuremethylester-Na-Salz | E 251 | Natriumnitrat |
| E 220 | Schwefeldioxid | E 252 | Kaliumnitrat |
| E 221 | Natriumsulfit | E 280 | Propionsäure |
| E 222 | Natriumyhdrogensulfit | E 281 | Natriumpropionat |
| E 223 | Natriumdisulfit | E 282 | Calciumpropionat |
| E 224 | Kaliumdisulfit | E 283 | Kaliumpropionat |
| E 226 | Calciumsulfit | E 290 | Kohlendioxid |

Ferner vorteilhaft sind in der Kosmetik gebräuchliche Konservierungsmittel oder Konservierungshilfsstoffe, wie Dibromdicyanobutan (2-Brom-2-brommethylglutarodinitril), Phenoxyethanol, 3-lod-2-propinylbutylcarbamat, 2-Brom-2-nitro-propan-1,3-diol, Imidazolidinylharnstoff, 5-Chlor-2-methyl-4-isothiazolin-3-on, 2-Chloracetamid, Benzalkoniumchlorid, Benzylalkohol.

Die erfindungsgemäßen Zubereitungen können vorteilhaft ein oder mehrere erfindungsgemäße waschaktive anionische, kationische, amphotere und/oder nicht-ionische Tenside enthalten. Es ist besonders vorteilhaft das oder die erfindungsgemäßen waschaktiven Tenside aus der Gruppe der Tenside zu wählen, welche einen HLB-Wert von mehr als 25 haben, ganz besonders vorteilhaft sind solche, welchen einen HLB-Wert von mehr als 35 haben.

Besonders vorteilhafte waschaktive anionische Tenside im Sinne der vorliegenden Erfindung sind
Acylaminosäuren und deren Salze, wie
■ Acylglutamate, insbesondere Natriumacylglutamat
■ Sarcosinate, beispielsweise Myristoyl Sarcosin, TEA-lauroyl Sarcosinat, Natriumlauroylsarcosinat und Natriumcocoylsarkosinat,
Sulfonsäuren und deren Salze, wie
■ Acyl-isethionate, z.B. Natrium-/ Ammoniumcocoyl-isethionat,
■ Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat
sowie Schwefelsäureester, wie
■ Alkylethersulfat, beispielsweise Natrium-, Ammonium-, Magnesium-, MIPA-, TIPA-Laurethsulfat, Natriummyrethsulfat und Natrium C₁₂₋₁₃ Parethsulfat,
■ Alkylsulfate, beispielsweise Natrium-, Ammonium- und TEA- Laurylsulfat.

Besonders vorteilhafte waschaktive kationische Tenside im Sinne der vorliegenden Erfindung sind q uarternäre Tenside. Q uaternäre Tenside enthalten mindestens ein N-Atom, das mit 4 Alkyl- oder Arylgruppen kovalent verbunden ist. Vorteilhaft sind Benzalkoniumchlorid, Alkylbetain, Alkylamidopropylbetain und Alkyl-amidopropylhydroxysultain.

Besonders vorteilhafte waschaktive amphotere Tenside im Sinne der vorliegenden Erfindung sind
■ Acyl-/dialkylethylendiamine, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,

Besonders vorteilhafte waschaktive nicht-ionische Tenside im Sinne der vorliegenden Erfindung sind
■ Alkanolamide, wie Cocamide MEA/ DEA/ MIPA,
■ Ester, die durch Veresterung von Carbonsäuren mit Ethylenoxid, Glycerin, Sorbitan oder anderen Alkoholen entstehen,
■ Ether, beispielsweise ethoxylierte Alkohole, ethoxyliertes Lanolin, ethoxylierte Polysiloxane, propoxylierte POE Ether und Alkylpolyglycoside wie Laurylglucosid, Decylglycosid und Cocoglycosid.

Weitere vorteilhafte anionische Tenside sind
■ Taurate, beispielsweise Natriumlauroyltaurat und Natriummethylcocoyltaurat,
■ Ether-Carbonsäuren, beispielsweise Natriumlaureth-13 Carboxylat und Natrium PEG-6 Cocamide Carboxylat,
■ Phosphorsäureester und Salze, wie beispielsweise DEA-Oleth-10 Phosphat und Dilaureth-4 Phosphat,
■ Alkylsulfonate, beispielsweise Natriumcocosmonoglyceridsulfat, Natrium C₁₂₋₁₄ Olefin-sulfonat, Natriumlaurylsulfoacetat und Magnesium PEG-3 Cocamidsulfat.

Weitere vorteilhafte amphotere Tenside sind
■ N-Alkylaminosäuren, beispielsweise Aminopropylalkylglutamid, Alkylaminopropionsäure, Natriumalkylimidodipropionat und Lauroamphocarboxyglycinat.

Weitere vorteilhafte nicht-ionische Tenside sind Alkohole.

Weitere geeignete anionische Tenside im Sinne der vorliegenden Erfindung sind ferner
■ Acylglutamate wie Di-TEA-palmitoylaspartat und Natrium Caprylic/ Capric Glutamat,
■ Acylpeptide, beispielsweise Palmitoyl hydrolysiertes Milchprotein, Natrium Cocoyl hydrolysiertes Soja Protein und Natrium-/ Kalium Cocoyl hydrolysiertes Kollagen
sowie Carbonsäuren und Derivate, wie
■ beispielsweise Laurinsäure, Aluminiumstearat, Magnesiumalkanolat und Zinkundecylenat,
■ Ester-Carbonsäuren, beispielsweise Calciumstearoyllactylat, Laureth-6 Citrat und Natrium PEG-4 Lauramidcarboxylat,
■ Alkylarylsulfonate.

Weitere geeignete kationische Tenside im Sinne der vorliegenden Erfindung sind ferner
■ Alkylamine,
■ Alkylimidazole und
■ ethoxylierte Amine.

Weitere geeignete nicht-ionische Tenside im Sinne der vorliegenden Erfindung sind ferner Aminoxide, wie Cocoamidopropylaminoxid.

Es ist vorteilhaft im S inn der vorliegenden Erfindung, wenn der Gehalt an einem oder mehreren waschaktiven Tensiden in der kosmetischen und/oder dermatologischen Reinigungsemulsion aus dem Bereich von 1 bis 25 Gew.-%, ganz besonders vorteilhaft von 5 bis 20 Gew.-% gewählt wird, jeweils bezogen auf das Gesamtgewicht der Zubereitungen.

Emulsionen im Sinne der vorliegenden Erfindung können vorzugsweise mindestens eine UV-A-, UV-B- und/oder Breitbandfiltersubstanz enthalten. Die Emulsionen können, obgleich nicht notwendig, gegebenenfalls auch weitere organische und/oder anorganische Pigmente als UV-Filtersubstanzen enthalten, welche in der Wasser- und/oder der Ölphase vorliegen können.

Besonders vorteilhafte bei Raumtemperatur flüssige UV-Filtersubstanzen im Sinne der vorliegenden Erfindung sind Homomenthylsalicylat (INCl: Homosalate), 2-Ethylhexyl-2-cyano-3,3-diphenylacrylat (INCl: Octocrylene), 2-Ethylhexyl-2-hydroxybenzoat (2-Ethylhexylsalicylat, Octylsalicylat, INCl: Octyl Salicylate) und Ester der Zimtsäure, vorzugsweise 4-Methoxyzimtsäure(2-ethylhexyl)ester (2-Ethylhexyl-4-methoxycinnamat, INCl: Octyl Methoxycinnamate) und 4-Methoxyzimtsäureisopentylester (Isopentyl-4-methoxycinnamat, INCl: Isoamyl p-Methoxycinnamate).

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.

Vorteilhafte weitere UV-Filtersubstanzen im Sinne der vorliegenden Erfindung sind sulfonierte, wasserlösliche UV-Filter, wie z. B.:
- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCl-Bezeichnung Bisimidazylate (CAS-Nr.: 180898-37-7), welches beispielsweise unter der Handelsbezeichnung Neo Heliopan AP bei Haarmann & Reimer erhältlich ist;
- Salze der 2-Phenylbenzimidazol-5-sulfonsäure, wie ihr Natrium-, Kalium- oder ihr Triethanolammonium-Salz sowie die Sulfonsäure selbst mit der INCl Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Haarmann & Reimer erhältlich ist;
- 1,4-Di(2-oxo-10-sulfo-3-bornylidenmethyl)-benzol (auch: 3,3'-(1,4-Phenylendimethylene)-bis-(7,7-dimethyl-2-oxo-bicyclo-[2.2.1]hept-1-ylmethansulfonsäure) und dessen Salze (besonders die entsprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird. Benzol-1,4-di(2-oxo-3-bornylidenmethyl-10-sulfonsäure) hat die INCl-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;
- Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Triazinderivate, wie z. B.
- 2,4-Bis-{[4-(2-Ethyl-hexyloxy)-2-hydroxy]-phenyl}-6-(4-methoxyphenyl)-1,3,5-triazin (INCl: Aniso Triazin), welches unter der Handelsbezeichnung Tinosorb® S bei der CIBA-Chemikalien GmbH erhältlich ist;
- Diethylhexylbutylamidotriazon (INCl: Diethylhexylbutamidotriazone), welches unter der Handelsbezeichnung UVASORB HEB bei Sigma 3V erhältlich ist;
- 4,4',4"-(1,3,5-Triazin-2,4,6-triyltriimino)-tris-benzoësäure-tris(2-ethylhexylester), auch: 2,4,6-Tris-[anilino-(p-carbo-2'-ethyl-1'-hexyloxy)]-1,3,5-triazin (INCl: Ethylhexyl Triazone), welches von der BASF Aktiengesellschaft unter der Warenbezeichnung UVINUL® T 150 vertrieben wird.

Auch andere Triazinderivate können erfindungsgemäß vorteilhaft eingesetzt werden.

Auch Benztriazolderivate können erfindungsgemäß vorteilhaft eingesetzt werden, beispielsweise [2,4'-Dihydroxy-3-(2H-benzotriazol-2-yl)-5-(1,1,3,3-tetramethylbutyl)-2'-noctoxy-5'-benzoyl]diphenylmethan, 2,2' Methylen-bis-[6-(2H-benzotriazol-2-yl)-4-(methyl)-phenol], 2,2'-Methylene-bis-[6-(2H-benzotiazol-2-yl)-4-(1,1,3,3-trtramethylbutyl)phenol], 2-(2'-Hydroxy-5'-octylphenyl)-benzotriazol, 2-(2'-Hydroxy-3',5'-di-t-amylphenyl)benzotriazol und 2-(2'-Hydroxy-5'-methylphenyl)benzotriazol.

Besonders vorteilhafte Benzotriazolderivate im Sinne der vorliegenden Erfindung sind das 2,2'-Methylen-bis-(6-(2H-benzotriazol-2-yl)-4-(1,1,3,3-tetramethylbutyl)-phenol) (Methylen Bis-Benzotriazolyl Tetramethylbutylphenol) (Tinosorb® M, CIBA-Chemikalien GmbH) und 2-(2H-benzotriazol-2-yl)-4-methyl-6-[2-methyl-3-[1,3,3,3-tetramethyl-1-[(trimethylsilyl)oxy]disiloxanyl]-propyl]-phenol (CAS-Nr.: 155633-54-8) mit der INCI-Bezeichnung Drometrizole Trisiloxane.

Die weiteren UV-Filtersubstanzen können öllöslich oder wasserlöslich sein.

Vorteilhafte öllösliche UV-B- und/oder Breitband-Filtersubstanzen im Sinne der vorliegenden Erfindung sind z. B.:
■ 3-Benzylidencampher-Derivate, vorzugsweise 3-(4-Methylbenzyliden)campher, 3-Benzylidencampher;
■ 4-Aminobenzoesäure-Derivate, vorzugsweise 4-(Dimethylamino)-benzoesäure(2-ethylhexyl)ester, 4-(Dimethylamino)benzoesäureamylester;
■ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
■ sowie an Polymere gebundene UV-Filter.
■ (3-(4-(2,2-bis Ethoxycarbonylvinyl)-phenoxy)propenyl)-methoxysiloxan/Dimethylsiloxan - Copolymer welches beispielsweise unter der Handelsbezeichnung Parsol® SLX bei Hoffmann La Roche erhältlich ist.

Weitere erfindungsgemäß vorteilhaft zu verwendende Lichtschutzfiltersubstanzen sind das Ethylhexyl-2-cyano-3,3-diphenylacrylat (Octocrylen), welches von BASF unter der Bezeichnung Uvinul® N 539 erhältlich ist und Hydroxybenzophenone wie der 2-(4'-(Diethylamino)-2'-hydoxybenzoyl)-benzoesäurehexylester.

Besonders vorteilhafte Zubereitungen im Sinne der vorliegenden Erfindung, die sich durch einen hohen bzw. sehr hohen UV-A- und/oder UV-B-Schutz auszeichnen, enthalten neben der oder den erfindungsgemäßen Filtersubstanz(en) bevorzugt ferner weitere UV-A- und/oder Breitbandfilter, insbesondere Dibenzoylmethanderivate [beispielsweise das 4-(tert.-Butyl)-4'-methoxydibenzoylmethan], Phenylen-1,4-bis-(2-benzimidazyl)-3,3'-5,5'-tetrasulfonsäure und/oder ihre Salze, das 1,4-di(2-oxo-10-Sulfo-3-bornylidenmethyl)-Benzol und/oder dessen Salze und/oder das 2,4-Bis-{[4-(2-Ethylhexyloxy)-2-hydroxy]-phenyl}-6-(4-methoxyphenyl)-1,3,5-triazin, jeweils einzeln oder in beliebigen Kombinationen miteinander.

Die Liste der genannten UV-Filter, die im Sinne der vorliegenden Erfindung eingesetzt werden können, soll selbstverständlich nicht limitierend sein.

Vorteilhaft enthalten die erfindungsgemäßen Emulsionen die Substanzen, die UV-Strahlung im UV-A- und/oder UV-B-Bereich absorbieren, in einer Gesamtmenge von z. B. 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der Emulsionen, um kosmetische Tücher zur Verfügung zu stellen, die das Haar bzw. die Haut vor dem gesamten Bereich der ultravioletten Strahlung schützen.

Bevorzugte weitere anorganische Pigmente sind Metalloxide und/oder andere in Wasser schwerlösliche oder unlösliche Metallverbindungen, insbesondere Oxide des Titans (TiO₂), Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxide der entsprechenden Metalle sowie Abmischungen aus solchen Oxiden sowie das Sulfat des Bariums (BaSO₄).

Die Pigmente können vorteilhaft im Sinne der vorliegenden Erfindung auch in Form kommerziell erhältlicher öliger oder wässriger Vordispersionen zur Anwendung kommen. Diesen Vordispersionen können vorteilhaft Dispergierhilfsmittel und/oder Solubilisationsvermittler zugesetzt sein.

Die Pigmente können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, daß die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen a us A luminiumoxid (Al₂O₃), A luminiumhydroxid Al(OH)₃, b zw. A luminiumoxidhydrat (auch: Alumina, CAS-Nr.: 1333-84-2), Natriumhexametaphosphat (NaPO₃)₆, Natriummetaphosphat (NaPO₃)ₙ, Siliciumdioxid (SiO₂) (auch: Silica, CAS-Nr.: 7631-86-9), oder Eisenoxid (Fe₂O₃). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Geeignete Titandioxidpartikel und Vordispersionen von Titandioxidpartikeln sind beispielsweise unter folgenden Handelsbezeichnungen bei den aufgeführten Firmen erhältlich:

| **Handelsname** | **Coating** | **Hersteller** |
|---|---|---|
| MT-100TV | Aluminiumhydroxid / Stearinsäure | Tayca Corporation |
| MT-100Z | Aluminiumhydroxid / Stearinsäure | Tayca Corporation |
| Eusolex T-2000 | Alumina / Simethicone | Merck KgaA |
| Titandioxid T805 (Uvinul TiO₂) | Octyltrimethylsilan | Degussa |

In kosmetischen, dermatologischen oder pharmazeutischen Formulierungen werden röntgenamorphe Oxidpigmente als Verdickungs- und Thixotropierungsmittel, Fließhilfsmittel, zur Emulsions- und Dispersionsstabilisierung und als Trägersubstanz (beispielsweise zur Volumenerhöhung von feinteiligen Pulvern oder Pudern) eingesetzt. Bekannte und in der kosmetischen oder dermatologischen Galenik oftmals verwendete röntgenamorphe Oxidpigmente sind die Siliciumoxide des Typs Aerosil® (CAS-Nr. 7631-86-9. Aerosile®, erhältlich von der Gesellschaft DEGUSSA, zeichnen sich durch geringe Partikelgröße (z.B. zwischen 5 und 40 nm) aus, wobei die Partikel als kugelförmige Teilchen sehr einheitlicher Abmessung anzusehen sind. Makroskopisch sind Aerosile® als lockere, weiße Pulver erkenntlich. Im Sinne der vorliegenden Erfindung sind röntgenamorphe Siliciumdioxidpigmente besonders vorteilhaft, und unter diesen gerade solche des Aerosil®-Typs bevorzugt.

Vorteilhafte Aerosil®-Typen sind beispielsweise Aerosil® OX50, Aerosil® 130, Aerosil® 150, Aerosil® 200, Aerosil® 300, Aerosil® 380, Aerosil® MOX 80, Aerosil® MOX 170, Aerosil® C OK 8 4, A erosil® R 2 02, A erosil® R 8 05, A erosil® R 812, Aerosil® R 972, Aerosil® R 974, Aerosil® R976.

Die erfindungsgemäßen kosmetischen und/oder dermatologischen Pickering-Emulsionen können 0,1 bis 20 Gew.-%, vorteilhaft 0,5 bis 10 Gew.-%, ganz besonders bevorzugt 1 bis 5 Gew.-% röntgenamorphe Oxidpigmente enthalten.

Erfindungsgemäß vorteilhaft lassen sich große Mengen saurer Aluminium- und/oder Aluminium/Zirkoniumsalze stabil in die Emulsionen einarbeiten. Es können 5 bis 40 Gewichts-%, insbesondere 10 bis 20 Gewichts-% Aluminiumchlorhydrat und/oder Aluminium/Zirkoniumchlorhydrat stabil in die Emulsionen eingearbeitet werden. Hierbei beziehen sich die beschriebenen Konzentrationsbereiche auf die sogenannte Aktivgehalte der Antitranspirant-Komplexe: bei den Aluminium-Verbindungen auf wasserfreie Komplexe, bei den Aluminium/Zirkonium-Verbindungen auf wasser- und pufferfreie Komplexe. Als Puffer wird hier üblicherweise Glycin verwendet.

Die nachfolgende Auflistung vorteilhaft einzusetzender Antitranspirant-Wirker soll in keinster Weise einschränkend sein:
Aluminium-Salze (der empirischen Summenformel [Al₂(OH)ₘClₙ], wobei m+n=6):
   - Aluminium-Salze wie Aluminiumchlorid AlCl₃, Aluminiumsulfat Al₂(SO₄)₃
   - Aluminiumchlorhydrat [Al₂(OH)₅Cl] x H₂O
      Standard Al-Komplexe: Locron L (Clariant), Chlorhydrol (Reheis), ACH-303 (Summit), Aloxicoll L (Giulini).
      Aktivierte Al-Komplexe: Reach 501 (Reheis), AACH-324 (Summit)
   - Aluminiumsesquichlorhydrat [Al₂(OH)_{4,5}Cl_{1,5}] x H₂O
      Standard Al-Komplexe: Aluminum Sesquichlorohydrate (Reheis), ACH-308 (Summit), Aloxicoll 31 L (Giulini)
      Aktivierte Al-Komplexe: Reach 301 (Reheis)
   - Aluminiumdichlorhydrat [Al₂(OH)₄Cl₂] x H₂O
Aluminium-Zirkonium-Salze:
   - Aluminium/Zirkonium Trichlorhydrex Glycin [Al₄Zr(OH)₁₃Cl₃] x H₂O x Gly
      Standard Al/Zr-Komplexe: Rezal 33GP (Reheis), AZG-7164 (Summit), Zirkonal P3G (Giulini)
      Aktivierte Al/Zr-Komplexe: Reach AZZ 902 (Reheis), AAZG-7160 (Summit), Zirkonal AP3G (Giulini)
   - Aluminium/Zirkonium Tetrachlorhydrex Glycin [Al₄Zr(OH)₁₂Cl₄] x H₂O x Gly
      Standard Al/Zr-Komplexe: Rezal 36G (Reheis), AZG-368 (Summit), Zirkonal L435G (Giulini)
      Aktivierte Al/Zr-Komplexe: Reach AZP 855 (Reheis), AAZG-6313-15 (Summit), Zirkonal AP4G (Giulini)
   - Aluminium/Zirkonium Pentachlorhydrex Glycin [Al₈Zr(OH)₂₃Cl₅] x H₂O x Gly
      Standard Al/Zr-Komplexe: Rezal 67 (Reheis), Zirkonal L540 (Giulini)
      Aktivierte Al/Zr-Komplexe: Reach AZN 885 (Reheis)
   - Aluminium/Zirkonium Octachlorhydrex Glycin [Al₈Zr(OH)₂₀Cl₈] x H₂O x Gly

Ebenso von Vorteil können aber auch Glycin-freie Aluminium/Zirkonium-Salze sein.

Dabei soll die Verwendung der Antitranspirant-Wirker aus den Rohstoffklassen Aluminium- und Aluminium/Zirkonium-Salzen nicht auf die handelsüblichen zumeist wäßrigen Lösungen, wie z.B. Locron L (Clariant), beschränkt sein, sondern es kann auch von Vorteil sein, die ebenfalls handelsüblichen wasserfreien Pulver derselbigen Rohstoffe durch Einbringung in die beanspruchten Formulierungen zum Einsatz zu bringen, wie z.B. Locron P (Clariant).

Vorteilhaft kann auch die Verwendung von sogenannten Antitranspirant-Salz Suspensionen sein, bei denen pulverförmig vorliegende Aluminium- und Aluminium/Zirkonium-Salze in diversen Ölen dispergiert angeboten werden.

Desweiteren kann es aber auch von Vorteil sein, spezielle Aluminium- und Aluminium/Zirkonium-Salze zum Einsatz zu bringen, die zur Löslichkeitsverbesserung als Glykol-Komplexe angeboten werden.

Weitere vorteilhafte Antitranspirant-Wirker basieren anstelle von Aluminium bzw. Zirkonium auf anderen Metallen, wie z.B. Beryllium, Titan, Hafnium.

Dabei soll die Liste der verwendbaren Antitranspirant-Wirker aber nicht auf metallhaltige Rohstoffe begrenzt sein, sondern von Vorteil sind auch Verbindungen, die Nichtmetalle wie Bor enthalten sowie solche, die dem Bereich der organischen Chemie zuzurechnen sind, wie z.B. Anticholinergika.

Vorteilhaft sind in diesem Sinne auch Polymere, die sowohl metallhaltig als auch metallfrei sein können.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **Beispiel 1** | |
|---|---|
| **Bestandteil** | **Menge / Gew.-%** |
| Paraffinum Liquidum | 99,8 |
| Parfum | 0,2 |

| **Beispiel 2:** Mikroemulsion | |
|---|---|
| **Bestandteil** | **Menge / Gew.-%** |
| Wasser | 82,0 |
| Paraffinum Liquidum | 8,0 |
| Glycerin | 5,0 |
| Octylstearat | 2,0 |
| Glyceryl Stearate, Ceteareth-20, Ceteareth-12, Cetearyl Alcohol, Cetyl Palmitate | 1,5 |
| Phenoxyethanol, Methylparaben, Ethylparaben, Propylparaben, Butylparaben, Isobutylparaben | 0,5 |
| Parfum | 0,4 |
| Ceteareth-20 | 0,3 |
| Methylparaben | 0,3 |
| Summe: | 100,0 |

| **Beispiel 3:** Mikroemulsion | |
|---|---|
| **Bestandteil** | **Menge / Gew.-%** |
| Wasser | 75,0 |
| Paraffinum Liquidum | 0,5 |
| Glycerin | 7,0 |
| Octylstearat | 1,0 |
| Glyceryl Stearate, Ceteareth-20, Ceteareth-12, Cetearyl Alcohol, Cetyl Palmitate | 3,0 |
| Phenoxyethanol, Methylparaben, Ethylparaben, Propylparaben, Butylparaben, Isobutylparaben | 0,5 |
| Parfum | 2,0 |
| Ceteareth-20 | 10,0 |
| Methylparaben | 1,0 |
| Summe: | 100,0 |

| **Beispiel 4:** wäßrige Tränkungslösung | |
|---|---|
| **Bestandteil** | **Menge / Gew.-%** |
| Wasser | 96,89 |
| Butylenglykol | 1,0 |
| PEG-40 Hydrogenated Castor Oil | 0,8 |
| Phenoxyethanol, Methylparaben, Ethylparaben, Propylparaben, Butylparaben, Isobutylparaben | 0,65 |
| Kaliumsorbat | 0,3 |
| Parfum | 0,2 |
| Zitronensäure | 0,16 |
| Summe: | 100,0 |

| **Beispiel 5:** wäßrige Tränkungslösung | |
|---|---|
| **Bestandteil** | **Menge / Gew.-%** |
| Wasser | 95,0 |
| Butylenglykol | 1,0 |
| PEG-40 Hydrogenated Castor Oil | 1,0 |
| Phenoxyethanol,Methylparaben, Ethylparaben, Propylparaben, Butylparaben, Isobutylparaben | 1,5 |
| Kaliumsorbat | 0,5 |
| Parfum | 0,5 |
| Zitronensäure | 0,5 |
| Summe: | 100,0 |

| **Beispiel 6** | |
|---|---|
| **Bestandteil** | **Menge / Gew.-%** |
| Cyclomethicone | 65,5 |
| Dimethicone | 20,0 |
| Silikongum | 7,0 |
| Phenyltrimethylmethicone | 7,0 |
| Parfum | 0,5 |
| Summe: | 100,0 |

| **Beispiel 7:** alkoholische Tränkungslösung | |
|---|---|
| **Bestandteil** | **Menge / Gew.-%** |
| Ethanol | 60,0 |
| Wasser | 34,5 |
| Glycerin | 5,0 |
| Parfum | 0,5 |
| Summe: | 100,0 |

| **Beispiel 8:** alkoholische Tränkungslösung | |
|---|---|
| **Bestandteil** | **Menge / Gew.-%** |
| Ethanol | 60,0 |
| Wasser | 24,0 |
| Glycerin | 5,0 |
| Isopropylalkohol | 5,0 |
| Ethylenediamine | 1,0 |
| Dexpanthenol | 1,0 |
| Carbomer | 3,0 |
| Parfum | 0,5 |
| Farbstoff | 0,5 |
| Summe: | 100,0 |

| **Beispiel 9:** After Sun-/Hautpflege-Mikroemulsion | |
|---|---|
| **Bestandteil** | **Menge / Gew.-%** |
| Ceteth-15 | 6 |
| Glycerylisostearate | 2 |
| Cetyl Alkohol | 1 |
| Dicaprylyl Carbonate | 5 |
| Octyldodecanol | 3 |
| Cylomethicone | 1 |
| Butylene Glycol | 3 |
| Ethanol | 5 |
| DMDM Hydantoin | 0,6 |
| Octoxyglycerin | 1 |
| Antioxydantien | 0,5 |
| Parfuem, | 0,5 |
| Farbstoffe | 0,3 |
| Wasser | ad 100 |

| **Beispiel 10:** nicht fettende Körperpflegeemulsion | |
|---|---|
| **Bestandteil** | **Gew.-%** |
| Ceteareth-12 | 6 |
| Glyceryl Stearate | 3,5 |
| Cetyl Palmitate | 3 |
| Dicaprylyl Ether | 5 |
| Cycolmethicone | 3 |
| Phenyl Trimethicone | 1 |
| Paraffinwax | 2 |
| Glycerin | 7,5 |
| Parabene | 1 |
| Phenoxyethanol | 1 |
| AGR | 0,5 |
| Parfuem | 0,5 |
| Farbstoffe | 0,5 |
| Wasser | ad 100 |

| **Beispiel 11:** Sonnenschutzmittel für seidiges Hautgefühl | |
|---|---|
| **Bestandteil** | **Gew.-%** |
| Ceteareth-20 | 5,5 |
| Glyceryl Stearate | 4 |
| Stearyl Alkohol | 3 |
| Dicaprylyl Ether | 5 |
| Octyldodecanol | 3 |
| Phenyl Trimethicone | 1 |
| Bis-Ethylhexyloxyphenol Methoxyphenyl Triazine | 2 |
| Octocrylene | 7 |
| Diethylhexyl Butamido Triazone | 1 |
| Ethylhexyl Methoxycinnamate | 4 |
| Butylene Glycol | 1 |
| Vitamin E Acetat | 1 |
| PVP/Hexadecene Copolymer | 1 |
| Parabene | 1 |
| Antioxydantien | 0,5 |
| Parfuem | 0,5 |
| Wasser | ad 100 |

| **Beispiel 12:** Sonnenschutzformulierung | |
|---|---|
| **Bestandteil** | **Gew.-%** |
| Ceteareth-20 | 6,5 |
| Glyceryl Stearate | 2 |
| Stearyl Alkohol | 1 |
| Dicaprylyl Carbonate | 5 |
| Octyldodecanol | 3 |
| C12-15 Alkyl Benzoate | 1 |
| Titandioxid | 2 |
| Bis-Ethylhexyloxyphenol Methoxyphenyl Triazine | 2 |
| Octocrylene | 7 |
| Ethylhexyl Methoxycinnamate | 4 |
| Parabene | 1 |
| Antioxydantien | 0,5 |
| Parfuem | 0,5 |
| Wasser | ad 100 |

| **Beispiel 13:** Sonnenschutzformulierung | |
|---|---|
| **Bestandteil** | **Gew.-%** |
| Steareth-20 | 6,5 |
| Glycerylisostearate | 2 |
| Cetyl Alkohol | 1 |
| Dicaprylyl Carbonate | 5 |
| Shea Butter | 3 |
| C12-15 Alkyl Benzoate | 1 |
| Bis-Ethylhexyloxyphenol Methoxyphenyl Triazine | 2 |
| Butylmethoxydibenzoylmethane | 1 |
| Ethylhexyl Triazone | 2 |
| Phenylbenzimidazol Sulfonsäure | 2 |
| Ethylhexyl Methoxycinnamate | 4 |
| Glycerin | 10 |
| Tricontanyl PVP | 1 |
| Citrat-Puffer | 1 |
| Parabene | 1 |
| Antioxydantien | 0,5 |
| Parfuem | 0,5 |
| Wasser | ad 100 |

| **Beispiel 14:** Sonnenschutzformulierung | |
|---|---|
| **Bestandteil** | **Gew.-%** |
| Ceteareth-30 | 7 |
| Glycerylisostearate | 2,5 |
| Cetyl Alkohol | 1 |
| Dicaprylyl Carbonate | 4 |
| Capric/Caprylic Triglyceride | 2 |
| C12-15 Alkyl Benzoate | 6 |
| Methylen Bis-Benzotriazolyl Tetramethylbutylphenol | 2 |
| Butyl Methoxydibenzoylmethane | 2 |
| Ethylhexyl Triazone | 4 |
| Bis-Imidazylat | 2 |
| Methylbenzylidene Camphor | 4 |
| Glycerin | 5 |
| PVP Hexadecene Copolymer | 1 |
| Parabene | 1 |
| Antioxydantien | 0,5 |
| Parfuem | 0,5 |
| Wasser | ad 100 |

| **Beispiel 15:** Sonnenschutzformulierung | |
|---|---|
| **Bestandteil** | **Gew.-%** |
| Ceteareth-20 | 7,5 |
| Glyceryl Stearate | 3 |
| Cetyl Palmitate | 1,5 |
| Dicaprylyl Carbonate | 5 |
| Cocoglycerides | 2 |
| C12-15 Alkyl Benzoate | 6 |
| Barium Sulfate | 2 |
| Bis-Ethylhexyloxyphenol Methoxyphenyl Triazine | 2 |
| Ethylhexyl Triazone | 4 |
| Bis-Imidazylat | 1 |
| Phenylbenzimidazol Sulfonsäure | 2 |
| Methylbenzylidene Camphor | 4 |
| PVP Hexadecene Copolymer | 1 |
| NaOH | 0,5 |
| Parabene | 1 |
| Antioxydantien | 0,5 |
| Parfuem | 0,5 |
| Wasser | ad 100 |

| **Beispiel 16:** After Sun-/Hautpflege-Formulierung | |
|---|---|
| **Bestandteil** | **Gew.-%** |
| Ceteth-15 | 6 |
| Glycerylisostearate | 2 |
| Cetyl Alkohol | 1 |
| Dicaprylyl Carbonate | 5 |
| Shea Butter | 1 |
| Octyldodecanol | 3 |
| Cylomethicone | 1 |
| Mineral Oil | 2 |
| Ethanol | 5 |
| Parabene | 1 |
| Antioxydantien | 0,5 |
| Parfuem | 0,5 |
| Wasser | ad 100 |

### Öl - Beispiele

| **Öl-1** | |
|---|---|
| **Bestandteil** | **Gew.-%** |
| Capric/Caprylic Triglyceride | 2 |
| C12-15 Alkyl Benzoate | 6 |
| Butyl Methoxydibenzoylmethane | 2 |
| Ethylhexyl Triazone | 2 |
| Bis-Ethylhexyloxyphenol Methoxyphenyl Triazine | 1 |
| Methylbenzylidene Camphor | 4 |
| Shea Butter | 1 |
| Butylene Glycol Dicaprate/Dicaprylate | 3 |
| Dimethicone | 5 |
| Parabene | 1 |
| Antioxydantien | 0,5 |
| Parfuem | 0,5 |
| Mineraloil | ad 100 |

| **Öl-2** | |
|---|---|
| **Bestandteil** | **Gew.-%** |
| Dicaprylyl Carbonate | 5 |
| Bis-Ethylhexyloxyphenol Methoxyphenyl Triazine | 2 |
| Ethylhexyl Triazone | 4 |
| Methylbenzylidene Camphor | 4 |
| Shea Butter | 1 |
| Octyldodecanol | 3 |
| Cylomethicone | 1 |
| Vitamin E | 1 |
| Parfuem | 0,5 |
| Mineraloil | ad 100 |

| **Öl-3** | |
|---|---|
| **Bestandteil** | **Gew.-%** |
| Dicaprylyl Carbonate | 5 |
| Bis-Ethylhexyloxyphenol Methoxyphenyl Triazine | 2 |
| Diethylhexyl Butamido Triazone | 4 |
| Methylbenzylidene Camphor | 1 |
| Shea Butter | 1 |
| Phenyltrimethicone | 1 |
| Vitamin E | 2 |
| Parfuem | 0,5 |
| Cyclomethicone | ad 100 |

| **Öl-4** | |
|---|---|
| **Bestandteil** | **Gew.-%** |
| Ethylhexyl Methoxycinnamate | 10 |
| Dicaprylyl Carbonate | 5 |
| Bis-Ethylhexyloxyphenol Methoxyphenyl Triazine | 2 |
| Diethylhexyl Butamido Triazone | 4 |
| Octocrylene | 5 |
| Shea Butter | 1 |
| Phenyltrimethicone | 1 |
| Vitamin E | 1 |
| Parfuem | 1 |
| Cyclomethicone | ad 100 |

| **Wäßrige Formulierung:** | |
|---|---|
| **Bestandteil** | **Gew.-%** |
| Bis-Imidazylat | 1 |
| Phenylbenzimidazol Sulfonsäure | 2 |
| Glycerin | 10 |
| Parabene | 1 |
| Antioxydantien | 0,5 |
| Parfuem | 0,5 |
| Wasser | ad 100 |

| **Hydrodispersion** | |
|---|---|
| **Bestandteil** | **Gew.-%** |
| Paraffinum Liquidum | 3,0000 |
| Glycerin | 1,0000 |
| VP/Hexadecene Copolymer | 0,5000 |
| Phenoxyethanol | 0,4000 |
| Parabene | 0,4000 |
| Parfum | 0,3000 |
| Acrylates/C10-30 Alkyl Acrylate Crosspolymer | 0,0500 |
| NaOH | 0,0200 |
| Wasser | ad 100 |

| **Sonnenhydrodispersion** | |
|---|---|
| **Bestandteile** | **Gew.-%** |
| BHT + Ethylhexyl Methoxycinnamate | 7,5000 |
| Glycerin | 5,0000 |
| Butylene Glycol Dicaprylate/Dicaprate | 5,0000 |
| Ethylhexyl Triazone | 4,0000 |
| Bis-Ethylhexyloxyphenol Methoxyphenyl Triazine | 2,5000 |
| Ethanol | 2,0000 |
| Trisodium EDTA | 1,0000 |
| C18-36 Acid Triglyceride | 1,0000 |
| Diethylhexyl Butamido Triazone | 1,0000 |
| Glyceryl Stearate Citrate | 1,0000 |
| Tocopheryl Acetate | 0,5000 |
| VP/Hexadecene Copolymer | 0,5000 |
| Phenoxyethanol | 0,4000 |
| DMDM Hydantoin | 0,4000 |
| Parfum | 0,3000 |
| NaOH | 0,1000 |
| Acrylates/C10-30 Alkyl Acrylate Crosspolymer | 0,2000 |
| Wasser | ad 100,0 |

## Patentansprüche

1. Kosmetischer Reinigungsartikel aus
c) einem Textil welches gebildet wird aus
1-30 Gewichts-% Baumwollfasern,
9-80 Gewichts-% Viskosefasern und
19-90 Gewichts-% Polyester, jeweils bezogen auf das Gesamtgewicht des Textils, welches **dadurch gekennzeichnet ist, dass** der Gehalt an Baumwollfasern im Textil zur Textiloberfläche hin zunimmt,
d) einer kosmetischen Zubereitung welche gewählt wird aus der Gruppe der Öle, Silikonöle, Emulsionen, Dispersionen, alkoholischen Lösungen und wässrigen Lösungen.

2. Reinigungsartikel nach Anspruch 1, **dadurch gekennzeichnet, dass** es sich bei dem Textil um ein Vlies handelt.

3. Reinigungsartikel nach einem der Ansprüche 1 oder 2, **dadurch gekennzeichnet, dass** das Textil eine durchschnittliche Dicke von 0,4 mm bis 2 mm aufweist.

4. Reinigungsartikel nach einem der Ansprüche 1 bis 3, **dadurch gekennzeichnet, dass** das Textil an der Oberfläche einen Baumwollanteil bis 30 Gewichts-% und im Inneren einen Baumwollanteil bis 10 Gewichts-%, jeweils bezogen auf das Gesamtgewicht des Textils, aufweist.

5. Reinigungsartikel nach einem der Ansprüche 1 bis 4, **dadurch gekennzeichnet, dass** der Tränkungsgrad, mit dem das Textil mit der kosmetischen Zubereitung getränkt ist, von 2,1 bis 4,0 beträgt.

6. Reinigungsartikel nach einem der Ansprüche 1 bis 5, **dadurch gekennzeichnet, dass** es sich bei der kosmetischen Zubereitung um ein Öl handelt, welches aus flüssigem Paraffin, Mineralöl, Cyclomethicon, Dimethicon, Silikongummi, Phenyltrimethylmethicon, Cariinsäure/Caprytsäure Triglycerid, C₁₂-C₁₅Alkylbenzoaten, Sheabutter, Butylenglycoldicaprat/-dicaprylat, Dicaprylylcarbonat, Octyldodecanol oder deren Mischungen gebildet wird.

7. Reinigungsartikel nach einem der Ansprüche 1 bis 5, **dadurch gekennzeichnet, dass** es sich bei der kosmetischen Zubereitung um eine Emulsion handelt, die Glycerylstearat und/oder Glycerylisostearat als Emulgator enthält.

8. Reinigungsartikel nach einem der Ansprüche 1 bis 5, **dadurch gekennzeichnet, dass** es sich bei der kosmetischen Zubereitung um eine Hydrodispersion handelt, die Glycerylstearatcitrat enthält.

9. Reinigungsartikel nach einem der Ansprüche 1 bis 5, **dadurch gekennzeichnet, dass** es sich bei der kosmetischen Zubereitung um eine alkoholische Lösung handelt, die mindestens 50 Gewichts%, bezogen auf das Gesamtgewicht der Zubereitung, Ethanol enthält.

10. Reinigungsartikel nach einem der Ansprüche 1 bis 5, **dadurch gekennzeichnet, dass** es sich bei der kosmetischen Zubereitung um eine wässrige. Lösung handelt, die Butylenglycol enthält.

11. Reinigungsartikel nach einem der vorhergehenden Ansprüche, **dadurch gekennzeichnet, dass** die kosmetische Zubereitung einen oder mehrere kosmetische oder dermatologische Wirk-, Hilfs- und Zusatzstoffe enthält.

## Claims

1. Cosmetic cleansing article composed of
c) a textile formed from 1-30% by weight of cotton fibres,
9-80% by weight of viscose fibres and
19-90% by weight of polyester, all based on the total weight of the textile,
said textile being **characterized in that** the content of cotton fibres in the textile increases in the direction of the textile surface,
d) a cosmetic preparation selected from the group consisting of oils, silicone oils, emulsions, dispersions, alcoholic solutions and aqueous solutions.

2. Cleansing article according to Claim 1, **characterized in that** the textile comprises a fibrous nonwoven web.

3. Cleansing article according to either of Claims 1 and 2, **characterized in that** the textile has an average thickness of 0.4 mm to 2 mm.

4. Cleansing article according to any one of Claims 1 to 3, **characterized in that** the textile has a cotton content up to 30% by weight at the surface and up to 10% by weight in its interior, both based on the total weight of the textile.

5. Cleansing article according to any one of Claims 1 to 4, **characterized in that** the saturation degree to which the textile is saturated with the cosmetic preparation is in the range from 2.1 to 4.0.

6. Cleansing article according to any one of Claims 1 to 5, **characterized in that** the cosmetic preparation comprises an oil formed from liquid paraffin, mineral oil, cyclomethicone, dimethicone, silicone rubber, phenyltrimethylmethicone, capric acid/caprylic acid triglyceride, C₁₂-C₁₅alkyl benzoates, shea butter, butylene glycol dicaprate/dicaprylate, dicaprylyl carbonate, octyldodecanol or mixtures thereof.

7. Cleansing article according to any one of Claims 1 to 5, **characterized in that** the cosmetic preparation comprises an emulsion containing glyceryl stearate and/or glyceryl isostearate as emulsifier.

8. Cleansing article according to any one of Claims 1 to 5, **characterized in that** the cosmetic preparation comprises a hydrodispersion containing glyceryl stearate citrate.

9. Cleansing article according to any one of Claims 1 to 5, **characterized in that** the cosmetic preparation comprises an alcoholic solution containing at least 50% by weight, based on the total weight of the preparation, of ethanol.

10. Cleansing article according to any one of Claims 1 to 5, **characterized in that** the cosmetic preparation comprises an aqueous solution containing butylene glycol.

11. Cleansing article according to any preceding claim, **characterized in that** the cosmetic preparation contains one or more active, excipient and adjunct cosmetic or dermatological ingredients.

## Revendications

1. Article de nettoyage cosmétique constitué par
c) un textile formé par
- 1-30% en poids de fibres de coton,
- 9-80% en poids de fibres de viscose et
- 19-90% en poids de polyester, à chaque fois par rapport au poids total du textile, **caractérisé en ce que** la teneur en fibres de coton augmente dans le textile vers la surface du textile,
d) une composition cosmétique qui est choisie dans le groupe des huiles, des huiles de silicose, des émulsions, des dispersions, des solutions alcooliques et des solutions aqueuses.

2. Article de nettoyage selon la revendication 1, **caractérisé en ce qu'**il s'agit, pour le textile, d'un non-tissé.

3. Article de nettoyage selon l'une quelconque des revendications 1 ou 2, **caractérisé en ce que** le textile présente une épaisseur moyenne de 0,4 mm à 2 mm.

4. Article de nettoyage selon l'une quelconque des revendications 1 à 3, **caractérisé en ce que** le textile présente à la surface une proportion de coton jusqu'à 30% en poids et à l'intérieur une proportion de coton jusqu'à 10% en poids, à chaque fois par rapport au poids total du textile.

5. Article de nettoyage selon l'une quelconque des revendications 1 à 4, **caractérisé en ce que** le degré d'imprégnation auquel le textile est imprégné avec la composition cosmétique est de 2,1 à 4,0.

6. Article de nettoyage selon l'une quelconque des revendications 1 à 5, **caractérisé en ce qu'**il s'agit, pour la composition cosmétique, d'une huile qui est formée par la paraffine liquide, l'huile minérale, la cyclométhicone, la diméthicone, le caoutchouc de silicone, la phényltriméthylméthicone, le triglycéride d'acide caprique/caprylique, les benzoates de C₁₂-C₁₅-alkyle, le beurre de karité, le dicaprate/dicaprylate de butylèneglycol, le dicaprylylcarbonate, l'octyldodécanol ou leurs mélanges.

7. Article de nettoyage selon l'une quelconque des revendications 1 à 5, **caractérisé en ce qu'**il s'agit, pour la composition cosmétique, d'une émulsion, qui contient du stéarate de glycéryle et/ou de l'isostéarate de glycéryle comme émulsifiant.

8. Article de nettoyage selon l'une quelconque des revendications 1 à 5, **caractérisé en ce qu'**il s'agit, pour la préparation cosmétique, d'une hydrodispersion, qui contient du stéarate-citrate de glycéryle.

9. Article de nettoyage selon l'une quelconque des revendications 1 à 5, **caractérisé en ce qu'**il s'agit, pour la composition cosmétique, d'une solution alcoolique, qui contient au moins 50% en poids, par rapport au poids total de la composition, d'éthanol.

10. Article de nettoyage selon l'une quelconque des revendications 1 à 5, **caractérisé en ce qu'**il s'agit, pour la préparation cosmétique, d'une solution aqueuse, qui contient du butylèneglycol.

11. Article de nettoyage selon l'une quelconque des revendications précédentes, **caractérisé en ce que** la composition cosmétique contient un(e) ou plusieurs substances actives, adjuvants et additifs.
